Europäisches Patentamt

European Patent Office

Office européen des brevets

(11) Publication number: **0 178 042**
A1

# EUROPEAN PATENT APPLICATION

(21) Application number: **85305164.7**

(22) Date of filing: **19.07.85**

(51) Int. Cl.⁴: **A 61 B 5/08**

(30) Priority: **19.07.84 IL 72446**

(43) Date of publication of application: **16.04.86**
**Bulletin 86/16**

(84) Designated Contracting States: **AT BE CH DE FR GB IT LI LU NL SE**

(71) Applicant: **MADEI SFIKA LTD., Aluf David Markus Street 10, Segula Petach Tikva (IL)**

(72) Inventor: **Grinbaum, Moshe, 37/16 Sharet Street, Hod Hasharon (IL)**

(74) Representative: **Arthur, Bryan Edward et al, Withers & Rogers 4 Dyer's Buildings Holborn, London EC1N 2JT (GB)**

(54) **Spirometer.**

(57) Spirometer apparatus including a housing (10) defining a fluid inlet (12) at one end thereof, a fluid outlet (14) at the other end thereof, and a flared diffuser portion (16); an aerodynamic reducer member (18) mounted within the diffuser portion (16) of the housing (10) and defining at least one, and preferably a plurality of fluid inlet apertures (20) and an outlet aperture congruent with the fluid outlet (14) of the housing; at least one oscillatory element (22), for example four, coupled to the fluid inlet apertures (20) of the aerodynamic member (18) and including a housing defining a fluid inlet and a fluid outlet, a cantilever member (23) pivotably mounted in the housing and arranged to be displaced in oscillatory motion in response to air flow there-along; and sensor apparatus (24) for sensing oscillation of the member and producing an output corresponding thereto.

## FIELD OF THE INVENTION

The present invention relates to spirometer apparatus for measuring expiratory air flow during breathing.

## BACKGROUND OF THE INVENTION

Many devices for measuring properties of a flow of air, commonly called spirometers, are known in the patent literature and in the marketplace. These include, inter alia, U.S. Patents 4,024,759, 4,425,803, 4,417,480 and 3,426,593. These conventional flow meters are generally designed to measure one of the following parameters: air volume, static pressure of air, or the flow rate of the air.

For the purposes of spirometry, or measurement of the expiratory air, the most widely used apparatus includes such devices as thermocouple gauge, turbine gauge with a piezoelectric or electromagnetic register element, and an acoustic gauge with a suspended sphere.

There is shown in U.S. Patent 4,312,235 to Daigle, a flowmeter for measuring the mass flow of a fluid stream in a duct including a sensor and electronic circuitry. The sensor includes a drag surface mounted on or forming part of an oscillatory element, which may comprise a cantilevered beam, mounted in the fluid stream for flow-damped oscillation. A drive inducer stimulates the oscillatory element and a pickup transducer senses the flow-damped oscillations. An intermediate circuit terminates the drive at a certain peak amplitude and measures the time

1

interval required for the amplitude of the flow-damped oscillations to decay a predetermined amount. That interval is indicative of the fluid mass flow.

These devices suffer from the disadvantage that they are generally incapable of measuring over a broad range of air flow rates or a range of volumes of air flow within a reasonable measuring time, i.e. without a high time lag. For example, most conventional spirometers have a response time of the order of 300 millisecs., while it is desired to measure in the time of 30 millisecs. Furthermore, most conventional spirometers can measure over a flow range which is only about half that desired by the medical profession today.

## SUMMARY OF THE INVENTION

It is therefore an object of the present invention to provide spirometer apparatus for measuring the flow rate and volume of air of a patient which overcomes the disadvantages of the prior art.

There is thus provided in accordance with the present invention spirometer apparatus including a housing defining a fluid inlet at one end thereof, a fluid outlet at the other end thereof, and a flared diffuser portion; an aerodynamic reducer member mounter within the diffuser portion of the housing and defining at least one, and preferably a plurality of fluid apertures and an outlet aperture congruent with the fluid outlet of the housing; at least one oscillatory element, for example four, coupled to the fluid inlet apertures of the aerodynamic

2

member and including a housing defining a fluid inlet and a fluid outlet, a cantilever member pivotably mounted in the housing and arranged to be displaced in oscillatory motion in response to air flow therealong; and sensor apparatus for sensing oscillation of the member and producing an output corresponding thereto.

According to a preferred embodiment, the spirometer further includes data processing apparatus coupled to the sensor apparatus, preferably including integrator apparatus for receiving the output from the sensor apparatus and providing an output corresponding to the volume of fluid flow.

Further according to a preferred embodiment, each of the oscillatory members includes a housing defining a fluid inlet and two fluid outlets, and two cantilever members pivotably mounted in the housing each arranged to be displaced in oscillatory motion in response to air flow therealong.

There is additionally provided in accordance with the present invention an oscillatory sensor member including a housing defining a fluid inlet and a fluid outlet, a cantilever member pivotably mounted in the housing and arranged to be displaced in oscillatory motion in response to air flow therealong, and sensor apparatus for sensing oscillation of the member and producing an output corresponding thereto.

According to one preferred embodiment, the sensor apparatus includes microphone apparatus. According to an alternative preferred embodiment, the sensor apparatus includes variable capacitor apparatus, the displaceable cantilever member comprising an element of the capacitor. It will also be

3

appreciated that all the optional features of the spirometer apparatus described herein relate correspondingly to the sensor apparatus of the invention per se, where applicable.

In a particular embodiment of the invention, the spirometer apparatus may include at least one further fluid outlet from the spirometer housing in the vicinity of the oscillatory element. Such an additional fluid outlet may be of practical application in the manufacture of the apparatus, as e.g. by injection molding. It does not adversely affect the operative use of the apparatus.

In a further aspect of the present invention, there is provided a spirometer apparatus as described above, except that the at least one oscillatory element comprises a plurality of oscillatory elements coupled to the at least one fluid inlet aperture of the aerodynamic reducer member and each such element comprises: a housing defining a fluid inlet and a fluid outlet; a cantilever member pivotably mounted in the housing and arranged to be displaced in oscillatory motion in response to air flow therealong; and sensor apparatus for sensing oscillation of at least one such cantilever member and producing a corresponding output, provided that at least one such oscillatory element comprises one or more cantilever members which is(are) devoid of such sensor means and act(s) as a gate to relieve patient discomfort when the air flow rate through the apparatus is in the higher end of the range. In this further aspect of the invention, it will be appreciated that all the optional features

4

of the spirometer apparatus described herein apply correspondingly.

## BRIEF DESCRIPTION OF THE DRAWINGS

The apparatus of the present invention will be further understood and appreciated from the following detailed description taken in conjunction with the drawings in which:

Fig. 1 is a sectional illustration of apparatus for measuring fluid flow constructed and operative in accordance with an embodiment of the present invention;

Figs. 2A and 2B are illustrations of an oscillatory member operative in the apparatus of the present invention in two different orientations during oscillation;

Fig. 3 is an illustration of an alternative oscillatory member operative in the apparatus of the present invention;

Fig. 4 is a block diagram illustration of a sensor apparatus circuit operative in the apparatus of the present invention; and

Fig. 5 is a block diagram illustration of an alternative sensor apparatus circuit operative in the apparatus of the present invention.

## DETAILED DESCRIPTION OF THE INVENTION

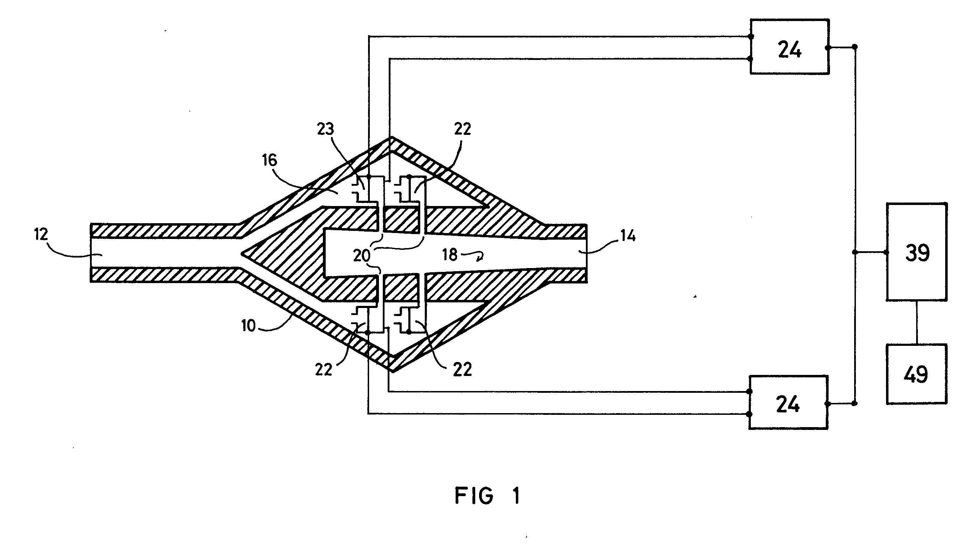

With reference to Fig. 1 there is shown spirometer apparatus constructed and operative in accordance with the present invention and comprising a housing 10 defining a fluid inlet 12, a fluid outlet 14 and a flared or widened middle portion, or diffuser 16. The spirometer of the present invention

5

0178042

is utilized for measuring the respiration of a patient, a human being or an animal. Thus, fluid inlet 12 may be shaped to form a convenient mouthpiece into which the patient breathes. This mouthpiece may be separately molded for easy disposal and replacement for each patient, or may be unitarily formed with the housing.

Mounted within housing 10 is a tapered aerodynamic reducer member 18 defining a closed member of continuously decreasing diameter having a fluid outlet at, and preferably coincident with, the fluid outlet 14 of the housing. Aerodynamic reducer member 18 may be of any shape defining cross-sections of continuously decreasing diameter along the air flow path, such as exponential, conical, gear conical profiles. Aerodynamic reducer member 18 serves to maintain the desired pressure drop across the housing, from inlet 12 to outlet 14. Housing 10 and aerodynamic reducer member 18 may be constructed of any suitable materials which meet the requirements of sterilizability, etc., of the medical profession.

It is a particular feature of the present invention that the reducer member together with the diffuser define an aerodynamic system which permits proper distribution of the air flow through the spirometer.

Aerodynamic reducer member 18 defines a plurality of fluid inlet apertures 20. Coupled to each inlet aperture 20 is the fluid outlet of an oscillatory element 22, including a cantilever member 23, which will be described in greater detail hereinbelow. Coupled to at least one oscillatory element 22 is

6

sensor means 24. (In the embodiment illustrated in Fig. 1 two oscillatory elements are so coupled and two are not; it is alternatively possible to have an arrangement whereby, for example, three oscillatory elements are coupled and one is not. The purpose of the one or more elements which are not so coupled is to act as gates so as to relieve patient discomfort when the air flow rate through the apparatus is in the higher end of the range.) Sensor means 24 may comprise any means for sensing oscillations of the cantilever member and providing an electrical output corresponding thereto. Preferably sensor 24 comprises a microphone, such as SM 81, manufactured by Shure, or electromagnetic circuit including a variable capacitor of which the cantilever member comprises one plate, the second plate being provided by the opposite wall of the oscillatory element.

The device illustrated in Fig. 1 may have an additional fluid outlet to the atmosphere (not shown), which is provided principally, as already stated, as an aid to manufacture by, for example, injection molding.

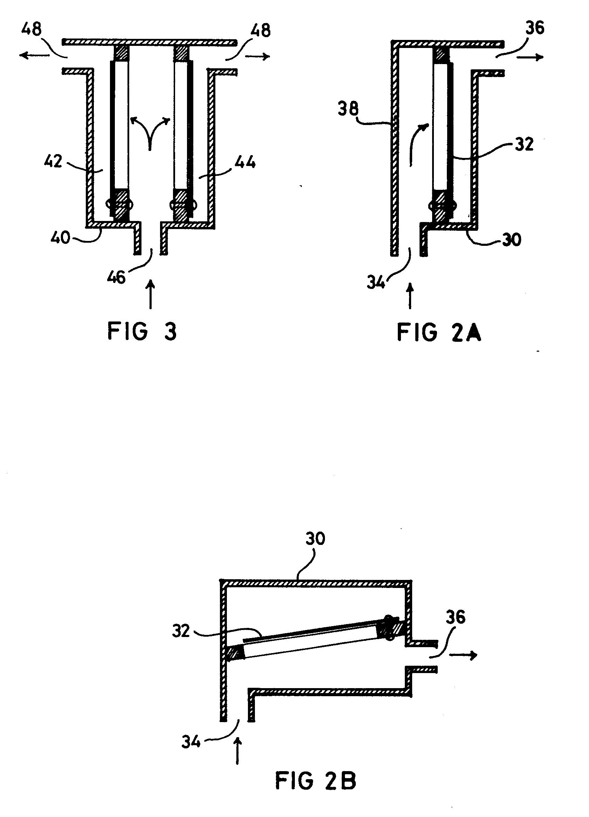

Referring now to Figs. 2A and 2B, there is shown an oscillatory element constructed and operative in accordance with the present invention and comprising a housing 30 defining an air inlet 34 and an air outlet 36, and a cantilever member 32 pivotably mounted in the housing. Cantilever member 32 is pivotably mounted in housing 30 closing the air outlet and is arranged to be displaced in oscillatory motion in response to air flow along the length of the member, thereby changing the outgoing air flow resistance.

7

It is a particular feature of the present invention that, inside the oscillatory element, the air flow is generally parallel to cantilever member 32 directly causing oscillation of the cantilever member. Thus, the oscillations generated by the air flow are measured directly with very little time lag. On the other hand, in existing flow meters including a cantilever element, as in U.S. Patent 4,312,235, oscillations are produced by a generator or drive transducer, and only flow damped oscillations of the cantilevered member, due to air flow perpendicular thereto, can be measured.

The dimensions of the housing 30 of each oscillatory member are such as to correspond with the resonance conditions of the volume of air at the frequency of fundamental cantilever vibration. The cross-sectional area of the fluid inlet 34 and outlet 36 determine the dynamic range in terms of flow rate of the oscillatory element, and is designed in accordance with the aerodynamic laws so as to permit inflow of the minimal air flow rate necessary for vibration of the particular cantilever member mounted therein. Thus, each cantilever element begins to oscillate only at a certain dynamic pressure drop depending on the flow rate of the incoming air. The maximum flow rate of the inflowing air for each cantilever element will be limited by the dimension of the outlet 36.

It is a particular feature of the present invention that the employment of a plurality of sensor-coupled oscillatory elements, each having a different dynamic threshold for vibration, permits the measurement of flow rate over a broad

8

range of rates. It is not possible to achieve this with the necessary accuracy and sensitivity with conventional spirometers. Integration of the sensed flow rates over time, as by any conventional integrator means 39, provides a measurement of volume of fluid flow through the apparatus for use by the physician.

Sensor apparatus is associated with the oscillatory member of Figs. 2A and 2B for sensing the oscillations of the cantilever member 32 in response to air flow. According to one preferred embodiment of the invention, the sensor apparatus comprises a microphone located within the housing of the spirometer for receiving the sound produced by the sonic oscillations of the cantilever members. The difference in resonance oscillatory frequency of each element permits determination of which element is oscillating which corresponds to the air flow rate.

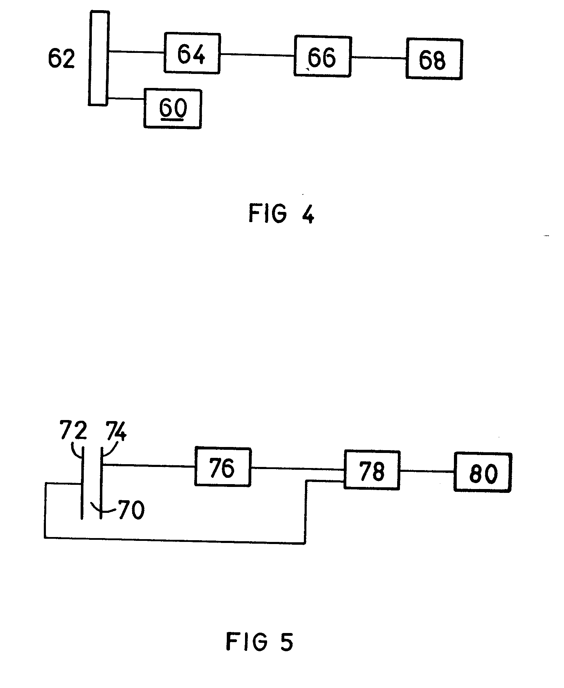

A block diagram illustration of a sensor circuit comprising a microphone 62 constructed and operative in accordance with an embodiment of the invention is shown in Fig. 4. Sonic oscillations generated by the cantilever member are received by microphone 62 which is coupled to a standard circuit. A power supply 60, such as a 1.5 volt battery, is provided to drive the microphone 62, for example SM 81 of Shure. An amplitude detector 66, such as one based on IC operational amplifier type UA 741 of Signetics, is provided to receive the output of an amplifier 64, for example, based on IC Operational Amplifier type 536 of Signetics, and is coupled to an integrator 68, such as one based on IC Operational Amplifier type 536 of Signetics, which

9

converts this information to a sensible output.

Alternatively according to a preferred embodiment, the sensor apparatus may comprise a variable capacitor, which can be understood with further reference to Fig. 2A and with reference to Fig. 5. Cantilevered member 32 serves as one plate of the capacitor while the rear wall 38 of housing 30 comprises the second plate. The amplitude of oscillation, which corresponds to the flow rate of the fluid, determines the maximal change of capacitance.

Fig. 5 illustrates a sensor circuit including a variable capacitor 70, one plate of which, 72, comprises the cantilever member of an oscillatory element, and the other plate 74 comprising the housing of the oscillatory element. A generator 76, for example type 555 IC Operational Amplifier of Signetics, is provided which generates electric oscillations with natural frequency. Oscillation of the cantilever member leads to modulation in capacity, followed by variations in the natural frequency of the generator which are measured by a detector 78, such as FM Demodulator (560B) of Signetics, which provides an output corresponding thereto to an integrator 80. These variations in frequency are proportional to the oscillation amplitude of the cantilever member which, in turn, corresponds to the rate of fluid flow.

Alternatively, the sensor apparatus may comprise an inducer coil. In this case, the cantilever member serves as a mobile element of inductivity instead of conductivity.

With reference to Fig. 3 there is shown an oscillatory

10

element constructed in accordance with an alternate embodiment of the invention, known as a tuning fork. This member comprises a housing 40 defining a single fluid inlet 46, two fluid outlets 48, and first and second cantilever members 42 and 44. The operation of this oscillatory element is essentially identical to that of Fig. 2A. Inflowing air through inlet 46 acts to cause oscillation of the cantilever members from a first orientation of resistance to air flow, to a second orientation permitting air flow. This oscillation can be measured by any sensor apparatus. The inclusion of two cantilever members provides a broader measurable range to the oscillatory element.

As can be seen with further reference to Fig. 1, the oscillatory elements are mounted about aerodynamic reducer member 18 in such a way that their fluid inlets 34 are facing inlet 12 and their fluid outlets 36 are each coupled to a fluid inlet 20 of the aerodynamic reducer member. Thus, fluid flowing into the apparatus flows in inlet 12 and around the closed end of the aerodynamic reducer member 18 in the widened diffuser portion 16 of housing 10. The relation between the cross-sections of the diffuser portion 16 and the aerodynamic reducer member 18 are designed to depend directly on flow resistance, within a given tolerance, provided by the oscillatory elements 22.

The fluid flows into the inlet of the appropriately configured oscillatory element and passes through it to outlet 20, causing the cantilever member 23 to vibrate at its natural resonance frequency. It will be appreciated that the amplitude of these oscillations in any given range is proportional to the rate of air flow.

11

The vibration of cantilever members 23 is sensed by sensors 24, as discussed above, and converted to a sensible output corresponding to the flow rate of the fluid through the apparatus. This output may be received by integrator means 39, and/or other data processing means 49 to provide flow rate or volume information in the desired form.

The outlet of each oscillatory element is designed for a certain rate of flow, having a diameter corresponding to the maximal flow rate measurable by the given element. The diameter of inlet 12 and outlet 14 are chosen in order to keep the pressure drop of a given air volume range passing through the aerodynamic system (diffuser and reducer) at the level required to overcome the resistance to air flow, which would make it impossible for patients to blow through the device.

It is a particular feature of the present invention that it is possible to measure in a wide range of expiratory air flow, e.g. when there are four cantilevers, the range can be 0.1 to 800 liters per minute. This measuring over a wide volume range is made possible by utilizing several oscillatory elements working in parallel. Each of the oscillatory elements is sensitive to a given flow rate interval, and all the elements together cover the entire range to be measured. The selectivity of an oscillatory element to a given range is secured by the following mechanism: First, the upper limit is set by the proper selection of the diameter of the outlet from the cantilever element. This permits the air volume corresponding to the maximal flow rate in the interval measurable by the given element

12

0178042

to flow therethrough.  Then the minimum threshold of the air flow able  to  generate the oscillations of the cantilever  member  is created  by  the selection of the diameter of the  inlet  to  the element  which  will  provide  the pressure  drop  necessary  for excitation  of  oscillation of cantilever  corresponding  to  the initial  part  of  the  flow rate range  measured  by  the  given oscillatory element.

It  is  a particular feature of the spirometer  of  the present  invention that a very short response time  is  provided, due  to  the  high sensitivity and detection  capability  of  the cantilever member in the oscillatory element. Measurements of the order of 30.millisec can be made with this apparatus.

The  spirometer  illustrated in Fig.  1,  comprising 2 oscillatory  elements  of different  resonance  ranges,  has  the measuring  capability  required by the medical profession  today. However, it will be appreciated that the measurable range of flow rate  can  be increased by providing  more  oscillatory  elements within the housing.

It will be appreciated by those skilled in the art that the invention is not limited to what has been shown and described hereinabove merely by way of example.  Rather,  the scope of  the invention is limited solely by the claims which follow.

13

CLAIMS

1. Spirometer apparatus comprising:

a housing defining a fluid inlet at one end thereof, a fluid outlet at the other end thereof, and a flared diffuser portion;

a aerodynamic reducer member mounted within the diffuser portion of said housing and defining at least one fluid inlet aperture and an outlet aperture at the fluid outlet of said housing;

at least one oscillatory element coupled to said at least one fluid inlet aperture of said aerodynamic reducer member and comprising:

a housing defining a fluid inlet and a fluid outlet;

a cantilever member pivotably mounted in said housing and arranged to be displaced in oscillatory motion in response to air flow therealong; and

sensor means for sensing oscillation of said member and producing an output corresponding thereto.

2. Spirometer apparatus comprising:

a housing defining a fluid inlet at one end thereof, a fluid outlet at the other end thereof, and a flared diffuser portion;

a aerodynamic reducer member mounted within the diffuser portion of said housing and defining at least one fluid inlet aperture and an outlet aperture at the fluid outlet of said housing;

0178042

a plurality of oscillatory elements coupled to said at least one fluid inlet aperture of said aerodynamic reducer member and each such element comprising:

a housing defining a fluid inlet and a fluid outlet;

a cantilever member pivotably mounted in said housing and arranged to be displaced in oscillatory motion in response to air flow therealong; and

sensor means for sensing oscillation of at least one such cantilever member and producing an output corresponding thereto, provided that at least one such oscillatory element comprises one or more cantilever members which is(are) devoid of such sensor means and act(s) as a gate to relieve patient discomfort when the air flow rate through the apparatus is in the higher end of the range.

3.      Apparatus according to claim 1 or claim 2 and wherein said aerodynamic reducer member defines cross-sections of continuously decreasing diameter along the air flow path.

4.      Apparatus according to claim 1 or claim 2 and further comprising data processing means coupled to said sensor means.

5.      Apparatus according to claim 4 and wherein said data processing means comprises integrator means for receiving the output from the sensor means and providing an output corresponding to the volume of fluid flow.

6.      Apparatus according to claim 1 or claim 2 and wherein

- 15 -

said oscillatory elements each comprise:

a housing defining a fluid inlet and two fluid outlets; and

two cantilever members mounted in the housing each arranged to be displaced in oscillatory motion in response to air flow therealong.

7. Spirometer apparatus according to claim 1 or claim 2 and wherein said at least one oscillatory element comprises a plurality of oscillatory elements.

8. Apparatus according to claim 1 or claim 2 and including at least one further fluid outlet from the spirometer housing in the vicinity of the oscillatory element.

9. An oscillatory sensor element comprising:

a housing defining a fluid inlet and a fluid outlet;

a member pivotably mounted in said housing and arranged to be displaced in oscillatory motion in response to air flow therealong; and

sensor means for sensing oscillation of said member and producing an output corresponding thereto.

10. Apparatus according to any of the preceding claims and wherein the diameters of the fluid inlet of each oscillatory element and the fluid outlet of that oscillatory element are selected such as to determine the upper and lower limits of the measurable air flow range in the element.

11. Apparatus according to any of the preceding claims and wherein said sensor means comprises microphone means.

12.     Apparatus according to any of the preceding claims and wherein said sensor means comprises electromagnetic sensor means.

13.     Apparatus according any of the preceding claims and wherein said sensor means comprises variable capacitor means, the cantilever member comprising an element of the capacitor.

14.     Apparatus according to any of the preceding claims and wherein said sensor means comprises an inducer coil, the cantilever member comprising a mobile element of inductivity.

15.     Spirometer apparatus substantially as described hereinbefore.

16.     Spirometer apparatus substantially as described hereinbefore with reference to the accompanying drawings.

17.     An oscillatory sensor element substantially as described hereinbefore.

18.     An oscillatory sensor element substantially as described hereinbefore with reference to the accompanying drawings.

0178042

FIG 1

0178042

2/3

FIG 3

FIG 2A

FIG 2B

62 | 64 | 66 | 68

60

FIG 4

72 74

76 | 78 | 80

70

FIG 5

European Patent Office

**EUROPEAN SEARCH REPORT**

Application number

| | DOCUMENTS CONSIDERED TO BE RELEVANT | | EP 85305164.7 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,Y<br>A | US - A - 4 312 235 (DAIGLE)<br>  * Abstract; column 2, lines 43-68; column 5, lines 29-62; fig. 1,2 *<br>-- | 1,2,9<br>4 | A 61 B 5/08 |
| Y<br>A | DE - A1 - 2 924 675 (BAUER)<br>  * Page 1, claim 1; page 2, claims 5,6; page 8, lines 18-21; fig. 1-3,7,8 *<br>-- | 1,2,9<br>6,7,12 | |
| A | DE - A1 - 2 404 318 (BOSCH)<br>  * Fig. 1,6 *<br>-- | 1,2,9,<br>12,14 | |
| Y | DE - B - 1 153 486 (VYZKUMNY A ZKUS-EBNI LETECKY USTAV)<br>  * Fig. *<br>-- | 1,2,9 | |
| D,Y<br>A | US - A - 3 426 593 (JACOBS)<br>  * Abstract; column 2, lines 37-58; fig. 1,2 *<br>-- | 1,2,9<br>4 | TECHNICAL FIELDS SEARCHED (Int. Cl.4)<br><br>A 61 B |
| A | EP - A2 - 0 091 522 (THE HOSPITAL FOR SICK CHILDREN)<br>  * Abstract; page 4, lines 6-16; fig. 1,3 *<br>-- | 1,2,4,<br>9,13 | |
| Y | DE - A1 - 3 020 265 (RESEARCH DE-VELOPMENT)<br>  * Fig. 1,3,4 *<br>-- | 1,2,9 | |

The present search report has been drawn up for all claims

| Place of search | Date of completion of the search | Examiner |
|---|---|---|
| VIENNA | 16-12-1985 | NEGWER |

**European Patent Office**

## EUROPEAN SEARCH REPORT

| | | | -2- |
|---|---|---|---|
| **DOCUMENTS CONSIDERED TO BE RELEVANT** | | | EP 85305164.7 |

| Category | Citation of document with indication, where appropriate, of relevant passages | Relevant to claim | CLASSIFICATION OF THE APPLICATION (Int. Cl.4) |
|---|---|---|---|
| Y | <u>GB - A - 1 554 408</u> (LUCAS INDU-STRIES) | 1,2,9 | |
| A | * Page 1, lines 69-75; page 2, lines 6-30; fig. 1 * | 3,4,10, 12,14 | |
| | -- | | |
| A | <u>GB - A - 892 348</u> (VYZKUMNY A ZKUS-EBNI LETECKY USTAV) | 1,2,9 | |
| | * Fig. 1 * | | |
| | -- | | |
| Y | <u>DD - A - 144 716</u> (SCITEC CORP.) | 1,2,9 | |
| A | * Abstract; page 8, paragraph 2; fig. 2,7 * | 3,4,12, 14 | |
| | -- | | |
| Y | <u>DE - A1 - 2 849 122</u> (HAUNOLD) | 1,2,9 | |
| A | * Page 1, claims 1,2,4 * | 4,5,10 | **TECHNICAL FIELDS SEARCHED (Int. Cl.4)** |

The present search report has been drawn up for all claims

| Place of search | Date of completion of the search | Examiner |
|---|---|---|
| VIENNA | 16-12-1985 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82